# EUROPÄISCHE PATENTSCHRIFT

(11) **EP 3 167 291 B1**
(45) Veröffentlichungstag und Bekanntmachung des Hinweises auf die Patenterteilung: **03.10.2018**
(21) Anmeldenummer: 15739536.9
(22) Anmeldetag: 10.07.2015
(51) Int. Cl.: G01N 33/68

(54) **IDENTITÄT UND REINHEIT VON GEWEBEBIOPSATEN**
IDENTITY AND PURITY OF TISSUE BIOPSIES
IDENTITÉ ET PURETÉ DE BIOPSIES TISSULAIRES

(30) Priorität: 11.07.2014 DE 102014213571
(43) Veröffentlichungstag der Anmeldung: 17.05.2017
(73) Patentinhaber: TETEC Tissue Engineering Technologies AG, 72770 Reutlingen (DE)
(72) Erfinder: BENZ, Karin, 73037 Göppingen (DE); FREUDIGMANN, Christian, 72770 Reutlingen (DE); GAISSMAIER, Christoph, 72127 Kusterdingen (DE); HECKY, Jochen, 79112 Freiburg (DE); MOLLENHAUER, Jürgen, verstorben (DE)
(74) Vertreter: Patentanwälte Ruff, Wilhelm, Beier, Dauster & Partner mbB
(86) Internationale Anmeldenummer: PCT/EP2015/065782
(87) Internationale Veröffentlichungsnummer: WO 2016/005543

(56) Entgegenhaltungen:
- WO-A1-94/14070
- DICKINSON SALLY C ET AL: "Quantitative outcome measures of cartilage repair in patients treated by tissue engineering", TISSUE ENGINEERING, LARCHMONT, NY, US, Bd. 11, Nr. 1-2, 1. Januar 2005 (2005-01-01), Seiten 277-287, XP009186541, ISSN: 1076-3279
- HOLLANDER ANTHONY P ET AL: "Quantitative analysis of repair tissue biopsies following chondrocyte implantation. + Discussions", NOVARTIS FOUNDATION SYMPOSIUM, WILEY, CHICHESTER, GB, Bd. 249, 1. Januar 2003 (2003-01-01), Seiten 218-241, XP009186540, ISSN: 1528-2511, DOI: 10.1002/0470867973.CH16 [gefunden am 2008-10-07]
- DANIEL J KELLY ET AL: "Biochemical markers of the mechanical quality of engineered hyaline cartilage", JOURNAL OF MATERIALS SCIENCE: MATERIALS IN MEDICINE, KLUWER ACADEMIC PUBLISHERS, BO, Bd. 18, Nr. 2, 1. Februar 2007 (2007-02-01), Seiten 273-281, XP019481252, ISSN: 1573-4838, DOI: 10.1007/S10856-006-0689-2

### SEQUENCE LISTING

<110> TETEC Tissue Engineering Technologies AG
<120> Identität und Reinheit von Gewebebiopsaten
<130> P54161WO
<160> 2
<170> PatentIn version 3.3
<210> 1
   <211> 6
   <212> PRT
   <213> Künstliche Sequenz
<220>
   <223> CTX-II
<400> 1
   Glu Lys Gly Pro Asp Pro
   1               5
<210> 2
   <211> 10
   <212> PRT
   <213> Künstliche Sequenz
<220>
   <223> NTX-I
<400> 2
   Thr Gly Val Glu Gly Pro Lys Gly Asp Thr
   1               5                   10

## Beschreibung

### Anwendungsgebiet und Stand der Technik

Die Erfindung betrifft ein Verfahren zum in vitro Nachweis und/oder zur in vitro Bestimmung der Reinheit von Knorpelgewebe, ein Verfahren zur Herstellung einer Knorpelzellkultur, ein Verfahren zur Herstellung eines knorpelzellbeladenen Implantats, die Verwendung von Protease-resistenten Fragmenten von Kollagen Typ II und/oder Kollagen Typ I sowie die Verwendung eines Kits.

Zur Durchführung einer Matrix-gestützten Chondrozytentransplantation (MACT) ist es erforderlich, dass quantitative Angaben zur Identität und Reinheit des verwendeten Knorpelgewebes sowie der verwendeten Knorpelzellen (Chondrozyten) gemacht werden können, welche in die für die Implantation vorgesehenen Implantate bzw. Matrices eingehen sollen.

Bislang erfolgt der Nachweis der Identität des verwendeten Knorpelgewebes über den Entnahmeort. Dieses anatomische Vorgehen entbehrt jedoch eines Prüfbeleges der korrekten Entnahme und erlaubt nicht eine Quantifizierung des entnommenen Gewebematerials im Hinblick auf die Reinheit gegen Knochen und Bindegewebe.

Im Falle eines aus einem menschlichen oder tierischen Kniegelenk entnommenen Knorpelgewebes kann zum Beispiel eine Kontamination mit Bindegewebe, wie beispielsweise Synovia, und/oder subchondralem Knochen nicht ausgeschlossen werden. Außerdem ist die Identität des Knorpelgewebes in den Fällen fraglich, in denen die Beschaffenheit des Biopsates eine eindeutige anatomische Zuordnung nicht zulässt.

Grundsätzlich kann die Identität sowie die Reinheit von Knorpelbiopsaten immunhistologisch untersucht werden. Neben Problemen der praktischen Durchführbarkeit unter Reinraumbedingungen haben immunhistologische Untersuchungen jedoch den Nachteil, dass stets nur kleine Segmente des präparierten Knorpels beurteilbar sind, da histologische Schnittpräparate typischerweise eine Dicke < 10 µm aufweisen. Eine umfassender Identitäts- und Reinheitsnachweis, der das ganze Präparat umfasst, ist daher nicht möglich.

Die Validierung von Regeneratbiopsien durch Nachweis eines C-terminalen Telopeptids von Collagentyp I sowie eines α1(II)-CB11B-Epitops ist bekannt (Sally C. Dickinson et al.: "Quantitative Outcome Measures of Cartilage Repair in Patients Treated by Tissue Engineering", Tissue Engineering volume 11, no. 1/2, 2005 und Anthony P. Hollander et al.: "Quantitative analysis of repair tissue biopsies following chondrocyte implantation", Tissue Engineering of Cartilage and Bone. Wiley, Chichester (Novartis Foundation Symposium 249) p 218-233).

Weiterhin ist die Evaluierung von Collagen des Typs I und II im Hinblick auf deren Eignung als biochemischer Marker für die Beurteilung der mechanischen Qualität von regeneriertem Knorpelgewebe bekannt (Daniel J. Kelly et al.: "Biochemical markers of the mechanical quality of engineered hyaline cartilage", J Mater Sci: Mater Med (2007) 18:273-281).

Aus der WO 94/14070 A1 ist ein Verfahren zur Bestimmung des Knorpelabbaus bekannt, bei welchem monoklonale Antikörper zum Nachweis von α1(II)-CB11B-Epitopen eingesetzt werden.

### Aufgabe und Lösung

Der vorliegenden Erfindung liegt daher die Aufgabe zugrunde, ein Verfahren zum in vitro Nachweis und/oder zur in vitro Bestimmung der Reinheit von Knorpelgewebe bereitzustellen, welches aus dem Stand der Technik bekannte Nachteile vermeidet. Das Verfahren soll insbesondere eine zweifelsfreie Identitätsbestimmung sowie gleichzeitig eine Reinheitsbestimmung von Knorpelgewebe ermöglichen. Es ist weiterhin eine Aufgabe der Erfindung, ein Verfahren zur Herstellung einer Knorpelzellkultur sowie ein Verfahren zur Herstellung eines knorpelzellbeladenen Implantats bereitzustellen. Außerdem ist es eine Aufgabe der Erfindung, die Verwendung von Protease-resistenten Kollagenfragmenten sowie die Verwendung eines Kits zur Durchführung der vorstehend genannten Verfahren bereitzustellen.

Diese Aufgaben werden gelöst durch Verfahren gemäß den Ansprüchen 1, 13 und 14 sowie durch eine Verwendung gemäß Anspruch 15. Bevorzugte Ausführungsformen sind in den abhängigen Ansprüchen 2 bis 12 definiert. Der Wortlaut sämtlicher Ansprüche wird hiermit durch ausdrückliche Bezugnahme zum Inhalt der vorliegenden Beschreibung gemacht. Weitere Lösungsvorschläge sind in der Beschreibung offenbart.

Gemäß einem ersten Aspekt betrifft die Erfindung ein Verfahren zum in vitro Nachweis, d.h. zum in vitro Identifizieren, von Knorpelgewebe und/oder zur in vitro Bestimmung der Reinheit von Knorpelgewebe. Bei dem Knorpelgewebe handelt es sich vorzugsweise um hyalines, insbesondere artikuläres, Knorpelgewebe. Das Verfahren umfasst die folgenden Schritte:
a) Behandeln einer Gewebeprobe mit einer Protease und
b) Untersuchen der Protease-behandelten Gewebeprobe auf die Anwesenheit von Protease-resistenten Fragmenten von Kollagen Typ II und/oder von Protease-resistenten Fragmenten von Kollagen Typ I.

Mit anderen Worten wird die Protease-behandelte, d.h. Protease-verdaute, Gewebeprobe beim Durchführen von Schritt b) auf Protease-resistente, d.h. gegenüber einer Protease stabile, Kollagenfragmente untersucht, welche durch Abbau von Kollagen Typ II und/oder durch Abbau von Kollagen Typ I entstehen bzw. freigesetzt werden.

Beim Durchführen von Schritt a) wird die Gewebeprobe in der Regel verflüssigt, so dass die oben erwähnten Kollagenfragmente in der Regel in eine verflüssigte Gewebeprobe freigesetzt werden.

Kollagen Typ II stellt allgemein das Leitkollagen für Knorpelgewebe dar. Dieser Kollagentyp findet sich nicht in Bindegewebe, wie beispielsweise Synovia, oder Knochengewebe, wie beispielsweise subchondralem Knochengewebe. Sowohl Binde- als auch Knochengewebe können beispielsweise bei Gelenkbiopsien als kontaminierendes Fremdgewebe auftreten. Binde- und Knochengewebe enthalten jeweils Kollagen Typ I als Leitkollagen, welches wiederum nicht in Knorpelgewebe vorkommt.

Die Erfindung beruht nun auf der überraschenden Erkenntnis, dass Protease-resistente Fragmente von Kollagen Typ II und/oder Kollagen Typ I die Bestimmung der Identität sowie der Reinheit von Knorpelgewebe erlauben. Hierbei konnte insbesondere festgestellt werden, dass die Menge an freigesetzten Protease-resistenten Fragmenten von Kollagen Typ II proportional zu der in der Probe enthaltenen Knorpelmenge und die Menge an freigesetzten Protease-resistenten Fragmenten von Kollagen Typ I proportional zu der in der Probe enthaltenen Fremdgewebemenge ist. Dies wiederum erlaubt nicht nur ein bloßes Identifizieren von Knorpelgewebe, sondern bei Anwesenheit von Fremdgewebe auch ein quantifizierendes Identifizieren von Knorpelgewebe, d.h. ein Bestimmen des Reinheitsgrades von in der Probe nachgewiesenem bzw. identifiziertem Knorpelgewebe.

Wird im Rahmen des erfindungsgemäßen Verfahrens festgestellt, dass die gemäß Schritt b) untersuchte Gewebeprobe Protease-resistente Fragmente von Kollagen Typ II enthält, kann die Probe Knorpelgewebe zugeordnet werden. Wird gleichzeitig festgestellt, dass die Gewebeprobe keine Protease-resistenten Fragmente von Kollagen Typ I enthält, handelt es sich bei der untersuchten Probe um reines Knorpelgewebe. Mit anderen Worten identifiziert der Nachweis von Protease-resistenten Fragmenten von Kollagen Typ II bei gleichzeitiger Abwesenheit von Protease-resistenten Fragmenten von Kollagen Typ I reines Knorpelgewebe. Da Knorpelgewebe nur aus einem Zelltyp, nämlich den sogenannten Chondrozyten (Knorpelzellen), besteht (Mollenhauer J, Kuettner KE: Articular Cartilage. In: Principles of Orthopaedic Practice. 2nd edition. Eds: R. Dee, L.C. Hurst, M.A. Gruber, Stephen A. Kottmeier. New York, McGraw Hill, 1997), erlaubt das erfindungsgemäße Verfahren über eine gelungene Identifikation von Knorpelgewebe zudem eine eindeutige Identifikation von Chondrozyten.

Wird im Rahmen des erfindungsgemäßen Verfahrens festgestellt, dass die gemäß Schritt b) untersuchte Gewebeprobe neben Protease-resistenten Fragmenten von Kollagen Typ II auch Protease-resistente Fragmente von Kollagen Typ I enthält, so kann die Probe einem mit Fremdzellen und/oder Fremdgewebe kontaminierten Knorpelgewebe zugeordnet werden. Obendrein kann über die freigesetzten Mengen an Protease-resistenten Fragmenten von Kollagen Typ II und Kollagen Typ I der Reinheitsgrad der Probe in Bezug auf Knorpelgewebe bestimmt werden. Als Fremdzellen kommen hauptsächlich Fibroblasten, Endothelzellen und/oder Osteoblasten in Betracht. Als Fremdgewebe kommen die bereit erwähnten Binde- und Knochengewebe in Frage.

Wird schließlich im Rahmen des erfindungsgemäßen Verfahrens festgestellt, dass die gemäß Schritt b) untersuchte Gewebeprobe keine Protease-resistenten Fragmente von Kollagen Typ II enthält, kann die Anwesenheit von Knorpelgewebe ausgeschlossen werden.

Als besonderer Vorteil ist hervorzuheben, dass das erfindungsgemäße Verfahren ein gezieltes Identifizieren von Gewebeproben erlaubt, welche aufgrund ihres Reinheitsgrades in Bezug auf Knorpelgewebe in besonderer Weise als Ausgangsmaterial für eine erfolgreiche Knorpelzellkultivierung und insbesondere für eine erfolgreiche Knorpelzellimplantation, bevorzugt Matrixgestützte Chondrozytentransplantation (MACT), geeignet sind.

In einer bevorzugten Ausführungsform ist das erfindungsgemäße Verfahren zum in vitro Nachweis von Knorpelgewebe und zur in vitro Bestimmung der Reinheit von Knorpelgewebe vorgesehen.

Vorzugsweise wird beim Durchführen von Schritt b) die Protease-behandelte Gewebeprobe sowohl auf Protease-resistente Fragmente von Kollagen Typ II als auch auf Protease-resistente Fragmente von Kollagen Typ I untersucht.

Bei der Gewebeprobe handelt es sich in einer weiteren Ausführungsform um ein menschliches oder tierisches, vorzugsweise menschliches, Gewebebiopsat oder um ein Fragment eines solchen Gewebebiopsats.

In einer weiteren Ausführungsform handelt es sich bei der Gewebeprobe um ein Knorpelbiopsat oder ein Fragment davon. Von dem Ausdruck "Knorpelbiopsat" sollen im Sinne der vorliegenden Erfindung nicht nur Biopsate erfasst werden, welche ausschließlich aus Knorpelgewebe, d.h. aus reinem Knorpelgewebe, bestehen, sondern auch Biopsate, welche neben Knorpelgewebe andere Gewebetypen, insbesondere Bindegewebe, wie beispielsweise Synovia, und/oder Knochengewebe, wie beispielsweise subchondrales Knochengewebe, und/oder neben Knorpelzellen andere Zelltypen, wie beispielsweise Fibroblasten, Endothelzellen und/oder Osteoblasten, enthalten.

In einer weiteren Ausführungsform handelt es sich bei der Gewebeprobe um ein Gelenkbiopsat, insbesondere Kniegelenkbiopsat, oder um ein Fragment davon.

Die Gewebeprobe wird in einer weiteren Ausführungsform vor Durchführen von Schritt a) mechanisch gereinigt, insbesondere mit Hilfe eines Skalpells.

In einer weiteren Ausführungsform werden vor Durchführen von Schritt a) Bindegewebe (wie beispielsweise Synovia), superfizielles Knorpelgewebe, mineralisiertes Knorpelgewebe und/oder Knochengewebe, insbesondere subchondrales Knochengewebe, aus der Gewebeprobe entfernt. Eine solche Reinigung kann von Vorteil sein, wenn die Gewebeprobe ausgehend von einem sogenannten Knorpel-Knochen-Stanzbiopsat, insbesondere wie im folgenden Absatz beschrieben, bereitgestellt wird.

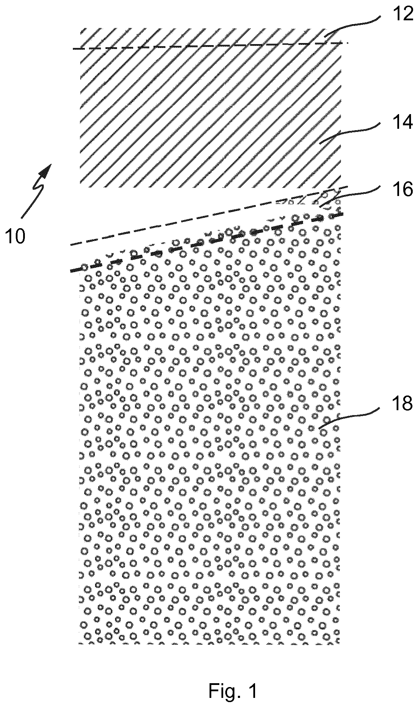

In einer weiteren Ausführungsform ist das Gewebebiopsat ein Gewebefragment, insbesondere ein Knorpelgewebefragment, eines Knorpel-Knochen-Stanzbiopsats. Das Stanzbiopsat ist in der Regel zylinderförmig und vorzugsweise einem menschlichen oder tierischen Kniegelenk entnommen. Ein solches Stanzbiopsat besitzt einen typischen anatomischen Aufbau, welcher sich aus einer superfiziellen Knorpelschicht, einer darunterliegenden mittleren Knorpelschicht, einer unter der mittleren Knorpelschicht liegenden mineralisierten Knorpelschicht sowie einer unter der mineralisierten Knorpelschicht liegenden subchondralen Knochenschicht zusammensetzt (siehe auch Figur 1). Bevorzugt entstammt das Gewebefragment der mittleren Knorpelschicht des Knorpel-Knochen-Stanzbiopsats.

In einer weiteren Ausführungsform wird die Gewebeprobe während eines Zeitraumes von 12 h bis 24 h, insbesondere 14 h bis 24 h, bevorzugt 19 h bis 24 h, mit der Protease behandelt.

Bei der Protease handelt es sich in einer weiteren Ausführungsform um Kollagenase, bevorzugt bakterielle Kollagenase. Eine geeignete Protease ist beispielsweise unter der Bezeichnung Liberase (Roche) kommerziell erhältlich.

In einer vorteilhaften Ausführungsform wird beim Durchführen von Schritt a) eine Lösung (sogenannte Verdaulösung) erhalten. Dies hat den Vorteil, dass eine homogene Verteilung der Protease-resistenten Fragmente von Kollagen Typ II und/oder Kollagen Typ I möglich ist, wodurch ein Aliquot einer solchen Lösung einen repräsentativen Querschnitt der zu untersuchenden Gewebeprobe darstellt. Auf diese Weise ist im Gegensatz zu immunhistologischen Untersuchungen ein umfassender Identitäts- und Reinheitsnachweis möglich.

Bei den Protease-resistenten Fragmenten von Kollagen Typ II und/oder Kollagen Typ I handelt es sich in einer weiteren Ausführungsform um quervernetzte Kollagenfragmente. Mit anderen Worten können die Protease-resistenten Fragmente von Kollagen Typ II und/oder Kollagen Typ I Quervernetzungen aufweisen. Die Quervernetzungen können gegebenenfalls frei von Peptidbindungen sein.

Die Quervernetzungen können insbesondere ausgewählt sein aus der Gruppe umfassend Allysin-Quervernetzungen, Hydroxyallysin-Quervernetzungen, Dehydro-hydroxylysino-norleucin-Quervernetzungen, Dehydro-lysino-norleucin-Quervernetzungen, Hydroxy-lysino-5-ketonorleucin-Quervernetzungen, Dihydroxy-lysino-norleucin-Quervernetzungen, Histidino-hydroxylysino-norleucin-Quervernetzungen, Hydroxylysylpyridinolin-Quervernetzungen, Lysylpyridinolin-Quervernetzungen, Pyridinium-Quervernetzungen, 3-Hydroxypyridinium-Quervernetzungen, Histidinohydroxymerodesmosin-Quervernetzungen und Kombinationen davon.

In einer bevorzugten Ausführungsform sind die Quervernetzungen ausgewählt aus der Gruppe umfassend Hydroxylysylpyridinolin-Quervernetzungen, Lysylpyridinolin-Quervernetzungen, Pyridinium-Quervernetzungen, 3-Hydroxypyridinium-Quervernetzungen und Kombinationen davon.

Die Protease-resistenten Fragmente von Kollagen Typ II und/oder Kollagen Typ I weisen in einer weiteren Ausführungsform 2 bis 15 Aminosäurereste, insbesondere 3 bis 10 Aminosäurereste, bevorzugt 4 bis 8 Aminosäurereste, auf.

Bevorzugt handelt es sich bei den Protease-resistenten Fragmenten von Kollagen Typ II und/oder Kollagen Typ I um N-terminale und/oder C-terminale Kollagenfragmente.

Um eine immunologische Diskriminierbarkeit (niedrige Kreuzreaktivität) und damit einen störungsfreien Nachweis der Protease-resistenten Kollagenfragmente zu erleichtern, handelt es sich bei den Protease-resistenten Fragmenten von Kollagen Typ II um C-terminale Kollagenfragmente und bei den Protease-resistenten Fragmenten von Kollagen Typ I um N-terminale Kollagenfragmente oder umgekehrt.

In einer besonders bevorzugten Ausführungsform handelt es sich bei den Protease-resistenten Fragmenten von Kollagen Typ II um C-terminale und vorzugsweise quervernetzte Telopeptide, d.h. um sogenannte C-terminale und vorzugsweise quervernetzte Typ II Kollagen Telopeptide (CTX-II), oder um Fragmente davon, d. h. C-terminale und vorzugsweise quervernetzte Typ II Kollagen Telopeptidfragmente (CTX-II-Fragmente). Die C-terminalen Telopeptide/ Telopeptidfragmente können insbesondere die Aminosäuresequenz EKGPDP bzw. eine Aminosäuresequenz gemäß SEQ ID NO: 1 gemäß beiliegendem Sequenzprotokoll aufweisen. Bezüglich der möglichen Quervernetzungen wird auf die bisherigen Ausführungen Bezug genommen.

In einer weiteren bevorzugten Ausführungsform handelt es sich bei den Protease-resistenten Fragmenten von Kollagen Typ I um N-terminale und vorzugsweise quervernetzte Telopeptide, d.h. um sogenannte N-terminale und vorzugsweise quervernetzte Typ I Kollagen Telopeptide (NTX-I) oder Fragmente davon, d. h. N-terminale und vorzugsweise quervernetzte Typ I Kollagen Telopeptidfragmente (NTX-I-Fragmente). Die N-terminalen Telopeptide/ Telopeptidfragmente können insbesondere die Aminosäuresequenz TGVEGPKGDT (UniProtKB/Swiss-Prot: P02452.5) bzw. eine Aminosäuresequenz gemäß SEQ ID NO: 2 gemäß beiliegendem Sequenzprotokoll aufweisen. Bezüglich der möglichen Quervernetzungen wird ebenso auf die bisherigen Ausführungen Bezug genommen.

Die Zusammensetzung und Struktur der in den beiden vorherigen Absätzen erwähnten C-terminalen und N-terminalen Telopeptide bzw. Telopeptidfragmente sind dem Fachmann an sich bekannt. Beispielhaft wird hingewiesen auf die folgenden Literaturstellen von Poole AR. "Immunochemical markers of joint inflammation, skeleton damage and repair: where are we now?" (Ann Rheum Dis. 1994 Jan; 53(1): 3-5), Garnero P, Piperno M, Gineyts E, Christgau S, Delmas PD, Vignon E. "Cross sectional evaluation of biochemical markers of bone, cartilage, and synovial tissue metabolism in patients with knee osteoarthritis: relations with disease activity and joint damage" (Ann Rheum Dis. 2001 Jun; 60(6):619-26), Herrmann M, Seibel MJ. "The amino- and carboxyterminal cross-linked telopeptides of collagen type I, NTX-I and CTX-I" (a comparative review. Clin Chim Acta. 2008 Jul 17; 292(2):57-75; Christgaue et al. Bone 2001; Garnero et al. "Association of Baseline Levels of Marker of Bone and Cartilage Degradation are associated with Longterm Progression of Joint Damage in Patients with Early Rheumatoid Arthritis: The Cobra Study" (Arthritis Rheum. 2002a 46(11): 2847-56); Garnero et al. "Uncoupling of type II collagen synthesis and degradation predicts progression of joint damage in patients with knee osteoarthritis" (Arthritis Rheum. 2002b 46(10): 2613-24); Christgau et al. "Collagen type II C-telopeptide fragments as an Index of cartilage degradation. Bone 2001; 29: 209-215) doi: 10.1016/j.cca.2008.03.020 Epub 2008 Mar 27 und Review. Erratum in: Clin Chim Acta. 2008 Nov; 397(1-2):103. Der Inhalt der vorstehend aufgezählten Literaturstellen wird bezüglich der darin beschriebenen C-terminalen und N-terminalen Telopeptide bzw. Telopeptidfragmente durch ausdrückliche Bezugnahme zum Inhalt der vorliegenden Beschreibung gemacht.

In einer bevorzugten Ausführungsform umfasst der Schritt b) des erfindungsgemäßen Verfahrens ein quantitatives Detektieren der Protease-resistenten Fragmente von Kollagen Typ II und/oder Kollagen Typ I, d.h. ein Bestimmen der in der Gewebeprobe enthaltenen Menge dieser Kollagenfragmente.

Bevorzugt wird der Grad der Reinheit der Gewebeprobe in Bezug auf darin enthaltenes Knorpelgewebes durch die prozentualen Mengenanteile an Protease-resistenten Fragmenten von Kollagen Typ II und/oder Typ I ermittelt.

In einer weiteren Ausführungsform wird zum Durchführen von Schritt b) die Protease-behandelte Gewebeprobe mit einem Antikörper oder Antikörperfragment kontaktiert, wobei der Antikörper bzw. das Antikörperfragment spezifisch gegen Protease-resistente Fragmente von Kollagen Typ II gerichtet ist. Auf diese Weise ist ein Detektieren, insbesondere ein quantitatives Detektieren, von derartigen Kollagenfragmenten in der Gewebeprobe, welche vorzugsweise verflüssigt vorliegt, möglich.

In einer weiteren Ausführungsform wird zum Durchführen von Schritt b) die Protease-behandelte Gewebeprobe mit einem Antikörper oder Antikörperfragment kontaktiert, wobei der Antikörper bzw. das Antikörperfragment spezifisch gegen Protease-resistente Fragmente von Kollagen Typ I gerichtet ist. Auf diese Weise ist ein Detektieren, insbesondere ein quantitatives Detektieren, von derartigen Kollagenfragmenten in der Gewebeprobe, welche vorzugsweise verflüssigt vorliegt, möglich.

Die verwendeten Antikörper bzw. Antikörperfragmente können monoklonal oder polyklonal sein. Die Herstellung derartiger Antikörper geschieht gemäß Standardverfahren, die dem Fachmann an sich bekannt sind und insbesondere durch Harlow und Lane 1988 und Köhler und Milstein 1975 in "Continuous cultures of fused cells secreting antibody of predefined specificity" (Nature 1975, 256 (5517): 495-7) sowie durch Campbell, A. M. Laboratory Techniques in Biochemistry and Molecular Biology, Band 13 (1986) beschrieben sind.

In einer weitergehenden Ausführungsform wird eine Bindung des Antikörpers bzw. Antikörperfragments an die Protease-resistenten Fragmente von Kollagen Typ II und/oder Kollagen Typ I mittels einer immunologischen Technik detektiert, insbesondere quantitativ detektiert. Die immunologische Technik ist vorzugsweise aus der Gruppe umfassend ELISA-Technik, immunoenzymatische Techniken, Immunofluoreszenz-Techniken, Radioimmunologie-Techniken, Chemoimmunologie-Techniken und Proteomanalyse-Techniken ausgewählt. Hierbei handelt es sich um medizinisch hinreichend etablierte und validierte, insbesondere auch CE-zertifizierte, Techniken.

Die Verwendung eines ELISA (Enzyme-Linked-Immunosorbent-Assay, Engvall, E. Meth Enzymol., 70, (1981)) ist erfindungsgemäß besonders bevorzugt. ELISA hat zum einen den Vorteil, dass es sich um ein unter labortechnischen Gesichtspunkten einfaches Verfahren handelt. Zum anderen ist von Vorteil, dass die mittels ELISA erzielbare Nachweisgrenze deutlich unterhalb der zur Durchführung des erfindungsgemäßen Verfahrens benötigten Empfindlichkeit liegt. So lassen sich beispielsweise mittels des erfindungsgemäßen Verfahrens 100 mg Gewebe, was einer typischen Probenmenge entspricht, in einer 14 ml Verdaulösung verarbeiten. Da Knorpel etwa 25 % vom Nassgewicht in Kollagen enthält, bedeutet dies ca. 25 mg kollagene Abbauprodukte. Hiervon machen die Protease-resistenten Fragmente von Kollagen Typ II und/oder Kollagen Typ I, insbesondere die zuvor erwähnten Telopeptide CTX-II oder NTX-I bzw. deren Fragmente, etwa 0.1 bis 1 % dieser Menge aus, also ca. 25 bis 250 mg. Verdünnt in 14 ml sind das also ungefähr 2 bis 20 µg/ml. Die Nachweisgrenzen eines ELISA liegen aber typischerweise im unteren bis mittleren Nanogramm/Milliliter-Bereich. Dadurch entsteht eine Nachweisgenauigkeit, die 100 bis 1000-fach über dem erforderlichen technischen Limit ist.

Gemäß einem zweiten Aspekt betrifft die Erfindung ein Verfahren zur Herstellung einer Knorpelzellkultur. Das Verfahren umfasst die folgenden Schritte:
a) Behandeln einer Knorpelgewebeprobe mit einer Protease,
b) Untersuchen der Protease-behandelten Knorpelgewebeprobe auf die Anwesenheit von Protease-resistenten Fragmenten von Kollagen Typ II und/oder Kollagen Typ I und
c) Kultivieren von in der untersuchten Knorpelgewebeprobe enthaltenen Knorpelzellen.

In einer bevorzugten Ausführungsform handelt es sich bei der Knorpelgewebeprobe um ein Knorpelbiopsat.

Insbesondere kann die Knorpelgewebeprobe aus einem Gelenkbiopsat, bevorzugt Kniegelenkbiopsat, stammen.

In einer weiteren Ausführungsform wird die Knorpelgewebeprobe vor Durchführen von Schritt a) mechanisch gereinigt, insbesondere mit Hilfe eines Skalpells.

In einer weiteren Ausführungsform werden vor Durchführen von Schritt a) Bindegewebe (wie beispielsweise Synovia), superfizielles Knorpelgewebe, mineralisiertes Knorpelgewebe und/oder Knochengewebe, insbesondere subchondrales Knochengewebe, aus der Knorpelgewebeprobe entfernt. Eine solche Reinigung kann insbesondere von Vorteil sein, wenn die Knorpelgewebeprobe aus einem Knorpel-Knochen-Stanzbiopsat stammt.

Die Knorpelgewebeprobe wird in einer weiteren Ausführungsform während eines Zeitraumes von 12 h bis 24 h, insbesondere 14 h bis 24 h, bevorzugt 19 h bis 24 h, mit der Protease behandelt.

In einer bevorzugten Ausführungsform umfasst der Schritt b) ein quantitatives Detektieren der Protease-resistenten Fragmente von Kollagen Typ II und/oder Kollagen Typ I, d. h. ein Bestimmen der in der Knorpelgewebeprobe enthaltenen Menge dieser Kollagenfragmente.

Bevorzugt wird der Grad der Reinheit des Knorpelgewebes durch die prozentualen Mengenanteile an Protease-resistenten Fragmenten von Kollagen Typ II und/oder Typ I ermittelt.

In einer weiteren Ausführungsform wird zum Durchführen von Schritt b) die Protease-behandelte Knorpelgewebeprobe mit einem Antikörper oder Antikörperfragment kontaktiert, wobei der Antikörper bzw. das Antikörperfragment spezifisch gegen Protease-resistente Fragmente von Kollagen Typ II gerichtet ist. Hierdurch ist ein Detektieren, insbesondere ein quantitatives Detektieren von derartigen Kollagenfragmenten in der Knorpelgewebeprobe möglich.

In einer weiteren Ausführungsform wird zum Durchführen von Schritt b) die Protease-behandelte Knorpelgewebeprobe mit einem Antikörper oder einem Antikörperfragment kontaktiert, wobei der Antikörper bzw. das Antikörperfragment spezifisch gegen Protease-resistente Fragmente von Kollagen Typ I gerichtet ist. Hierdurch ist ein Detektieren, insbesondere ein quantitatives Detektieren, von derartigen Kollagenfragmenten in der Knorpelgewebeprobe möglich.

Ein initiales Kultivieren der Knorpelzellen erfolgt vorzugsweise in einer sogenannten Suspensionskultur. Eine Suspensionskultur ist eine Zellkultur mit nicht-adhärenten Zellen. Die Zellen bleiben in Suspension, da sie keinen direkten Kontakt zu einem Substrat benötigen.

Bevorzugt werden die Knorpelzellen während eines Zeitraumes von 1 bis 3 Tagen, insbesondere von 2 Tagen, kultiviert.

Ein Behandeln der Knorpelgewebeprobe mit Protease in Kombination mit einer vorzugsweise mehrtägigen, insbesondere zweitägigen, Suspensionskultur ist besonders vorteilhaft, da sich herausstellte, dass weder Knochenzellen noch Endothelzellen eine derartige Etablierungsphase der Zellkultur überleben.

In einer weiteren Ausführungsform wird zum Durchführen von Schritt c) ein sogenanntes Expansionsmedium verwendet. Unter einem Expansionsmedium wird ein Zellkulturmedium verstanden, welches zur Zellvermehrung eingesetzt wird. Die Verwendung eines solchen Expansionsmediums hat den Vorteil, dass die Proliferationsgeschwindigkeit von gereinigten Fibroblasten und Osteoblasten nicht höher ist als die der Knorpelzellen und dass auch gereinigte Endothelzellen in einem solchen Proliferationsmedium absterben.

Bezüglich weiterer Merkmale und Vorteile des Verfahrens, insbesondere der Protease-resistenten Fragmente von Kollagen Typ II und/oder I, der Antikörper sowie geeigneter immunlogischer Techniken zum Durchführen von Schritt b), wird vollständig auf die im Rahmen des ersten Erfindungsaspektes gemachten Ausführungen Bezug genommen.

Gemäß einem dritten Aspekt betrifft die Erfindung ein Verfahren zur Herstellung eines knorpelzellbeladenen Implantats, insbesondere zur Verwendung bei der Matrix-gestützten Chondrozytentransplantation (MACT), umfassend die folgenden Schritte:
a) Behandeln einer Knorpelgewebeprobe mit einer Protease,
b) Untersuchen der Protease-behandelten Knorpelgewebeprobe auf die Anwesenheit von Protease-resistenten Fragmenten von Kollagen Typ II und/oder Kollagen Typ I,
c) Kultivieren von in der untersuchten Knorpelgewebeprobe enthaltenen Knorpelzellen und
d) Beladen eines Implantats mit den kultivierten Knorpelzellen.

Zur Beladung mit Knorpelzellen geeignete Implantate stellen insbesondere kollagenhaltige Implantate dar.

Bevorzugt wird zum Durchführen von Schritt d) ein schichtförmig aufgebautes Implantat aus einer membranförmigen Komponente sowie einer schwammförmigen Komponente verwendet, wobei die schwammförmige Komponente vorzugsweise säulenförmige Poren besitzt, welche insbesondere senkrecht oder im Wesentlichen senkrecht zur membranförmigen Komponente orientiert sind. Derartige Poren lassen sich bevorzugt mittels einseitiger Lyophilisierung ausbilden. Bevorzugt handelt es sich bei der membranförmigen Komponente um eine Perikardmembran und bei der schwammförmigen Komponente um einen Kollagenschwamm. Ein entsprechendes Implantat wird von der Anmelderin beispielsweise unter der Bezeichnung Novocart® Basic bzw. Novocart® 3D kommerziell vertrieben. Bezüglich weiterer Merkmale und Vorteile eines solchen Implantats wird zudem auf die EP 1 824 420 B1 Bezug genommen, deren Offenbarungsgehalt in Bezug auf das darin beschriebene Implantat zur Reparatur eines Knorpeldefektes hiermit durch ausdrückliche Bezugnahme zum Inhalt der vorliegenden Beschreibung gemacht wird.

Bezüglich weiterer Merkmale und Vorteile des Verfahrens wird vollständig auf die im Rahmen der bisherigen Erfindungsaspekte gemachten Ausführungen Bezug genommen.

Gemäß einem vierten Aspekt betrifft die Erfindung die Verwendung von Protease-resistenten Fragmenten von Kollagen Typ II und/oder Kollagen Typ I zum in vitro Nachweis von Knorpelgewebe und/oder zur in vitro Bestimmung der Reinheit von Knorpelgewebe.

Bezüglich weiterer Merkmale und Vorteile der Verwendung, insbesondere der Protease-resistenten Fragmente von Kollagen Typ II und/oder Kollagen Typ I, wird ebenfalls vollständig auf die im Rahmen der bisherigen Erfindungsaspekte gemachten Ausführungen Bezug genommen.

Gemäß einem fünften Aspekt wird ein Kit, vorzugsweise immunologischer Kit, zur Durchführung eines im Rahmen der bisherigen Erfindungsaspekte beschriebenen Verfahrens offenbart.

Das Kit umfasst wenigstens zwei Komponenten, welche ausgewählt sind aus der Gruppe umfassend Antikörper, Antikörperfragmente, Antikörper-Reporterenzym-Konjugate, Antikörperfragmente-Reporterenzym-Konjugate, Enzymsubstrate, insbesondere Farbsubstrate (Chromogene), Mikrotiterplatten, Pufferlösungen, Detergenzien, Proteinstabilisatoren, Konservierungsmittel und Kombinationen davon.

Die Reporterenzyme können ausgewählt sein aus der Gruppe umfassend Meerrettichperoxidase, alkalische Phosphatase und Glucose-Oxidase. Farbstoffsubstrate können ausgewählt sein aus der Gruppe umfassend para-Nitrophenylphosphat und o-Phenylendiamin.

Die oben genannten Pufferlösungen können bereits übliche Additive, wie beispielsweise Detergenzien, Proteinstabilisatoren und/oder Konservierungsmittel, enthalten.

Bezüglich weiterer Merkmale und Vorteile wird ebenso vollständig auf die im Rahmen der bisherigen Erfindungsaspekte gemachten Ausführungen Bezug genommen.

Weitere Merkmale und Vorteile der Erfindung ergeben sich aus den nachfolgend beschriebenen Ausführungsformen anhand von Figuren, Figurenbeschreibungen eines Beispiels sowie der Unteransprüche. Hierbei können einzelne Merkmale der Erfindung alleine oder in Kombination miteinander verwirklicht sein. Die beschriebenen Ausführungsformen dienen lediglich zur Erläuterung und zum besseren Verständnis der Erfindung und sind in keiner Weise einschränkend zu verstehen.

### Figurenkurzbeschreibungen

- Fig. 1: zeigt schematisch den anatomischen Aufbau einer Knochen-Knorpel-Stanze 10, welche aus einem Kniegelenk eines menschlichen Patienten entnommen ist. Bei der Schicht 12 handelt es sich um die sogenannte superfizielle Knorpelschicht. Die darunter liegende mittlere Knorpelschicht 14 ist für die eigentliche Bereitstellung des Knorpelgewebes sowie der Knorpelzellen vorgesehen. Darunter befindet sich eine mineralisierte Knorpelschicht 16, gefolgt von einer darunter liegenden subchondralen Knochenschicht 18. Da sich Knochen und mineralisierter Knorpel in der Regel nicht durchtrennen lassen, erfolgt eine Separation entlang der gestrichelten Linie, d. h. am Boden der nicht mineralisierten Knorpelschicht 14. Die gepunktete Linie zeigt den Verlauf der Knorpel-Knochen-Grenze an.

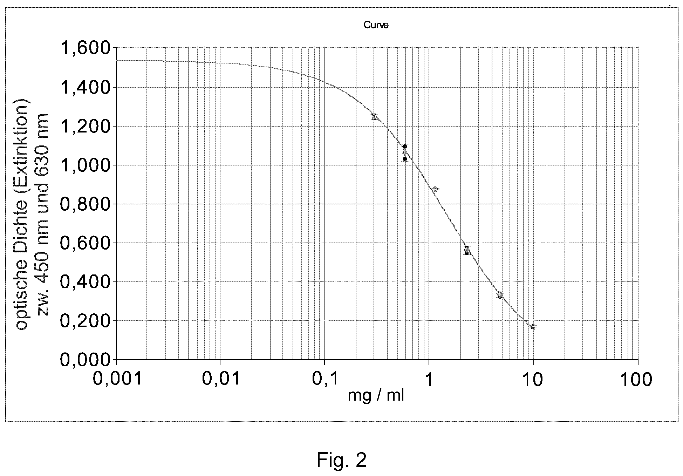

- Fig. 2: zeigt schematisch die Standardkurve eines für den Nachweis von CTX-II verwendeten ELISA (Urine CartiLaps® ELISA). Auf der Ordinate ist die optische Dichte (Extinktion) in einem Wellenlängenbereich von 450 nm bis 630 nm aufgetragen. Auf der Abszisse ist die Konzentration in mg/ml aufgetragen.

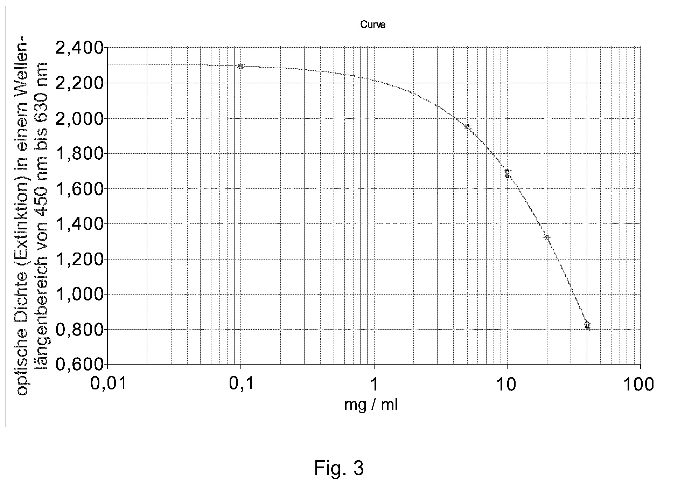

- Fig. 3: zeigt schematisch eine Standardkurve eines für den Nachweis von NTX-I verwendeten ELISA (OSTEOMARK® NTX-Serum ELISA). Auf der Ordinate ist die optische Dichte (Extinktion) in einem Wellenlängenbereich von 450 nm bis 630 nm aufgetragen. Auf der Abszisse ist die Konzentration in nM BCE (Bone Collagen Equivalents, Knochenkollagenäquivalente) aufgetragen.

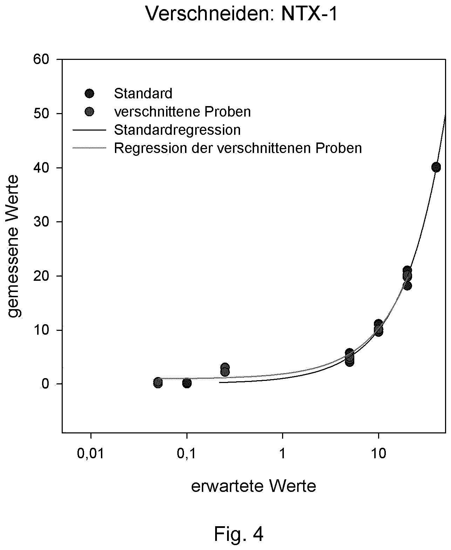

- Fig. 4: zeigt schematisch eine logarithmische Darstellung der regulären Standardkurve in Puffer sowie der Standardkurve in Gegenwart von Verdaulösung für NTX-I (1:50 Knorpelverdau). Auf der Ordinate ist die gemessene Konzentration in nM BCE/mm angegeben. Auf der Abszisse ist die erwartete Konzentration in nM BCE/mm angegeben. Die logarithmische Darstellung wurde gewählt, um die kleinen Konzentrationen angemessen darstellen zu können.

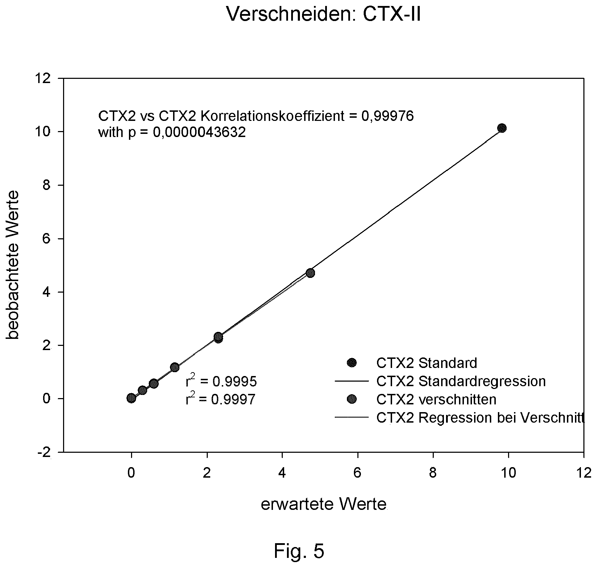

- Fig. 5: zeigt schematisch die Darstellung der regulären Standardkurve in Puffer sowie der Standardkurve in Gegenwart von Verdaulösung für CTX-II (1:1000 Synovialverdau). Auf der Ordinate ist die gemessene Konzentration in ng/ml angegeben. Auf der Abszisse ist die erwartete Konzentration in ng/ml angegeben.

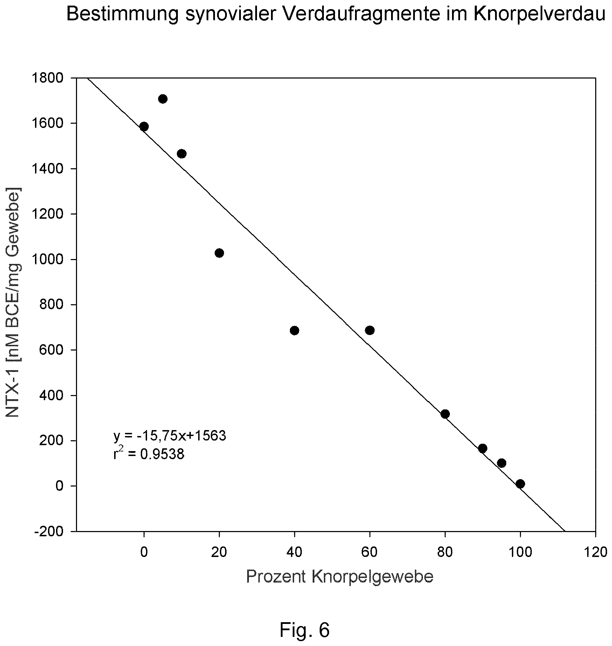

- Fig. 6: zeigt schematisch das Ergebnis der Bestimmung synovialer Verdaufragmente im Knorpelverdau. Dabei bedeutet "0 % Knorpelverdau" reinen Synovialverdau. Auf der Ordinate ist die Konzentration von NTX-I in nM BCE/mg Gewebe angegeben. Auf der Abszisse ist der Anteil an Knorpelverdau in % angegeben.

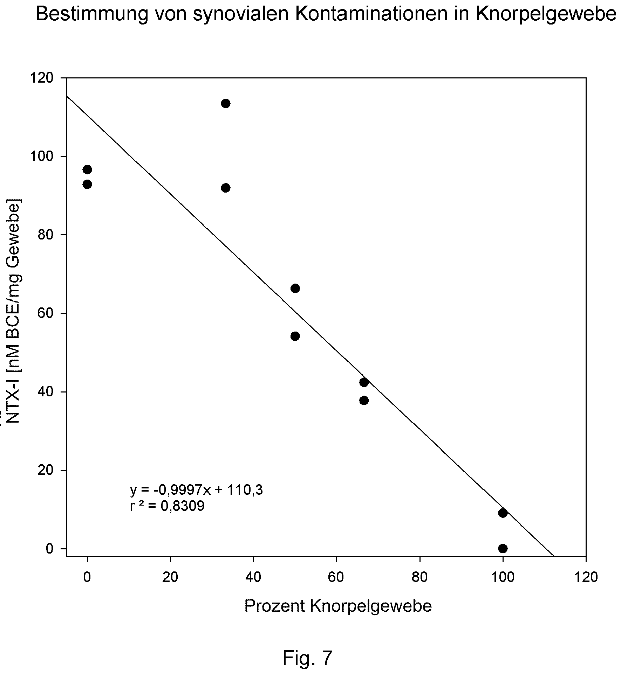

- Fig. 7: zeigt grafisch das Ergebnis eines Kontaminationsexperiments, bei dem Knorpelgewebe mit Synovialgewebe vermischt wurde. Die tatsächlichen Einwaagen sind: 100 % Synovia -115.7 mg Knorpel, 66.6 % Synovia -67.9 mg + 31.2 mg Knorpel, 50 % Synovia - 52.2 mg + 53.6 mg Knorpel und 33.3 % Synovia - 30.4 mg + 66.8 mg Knorpel. Auf der Ordinate ist die Konzentration von NTX-I in nM BCE/mg Gewebe dargestellt. Auf der Abszisse ist Knorpelgewebe in % dargestellt.

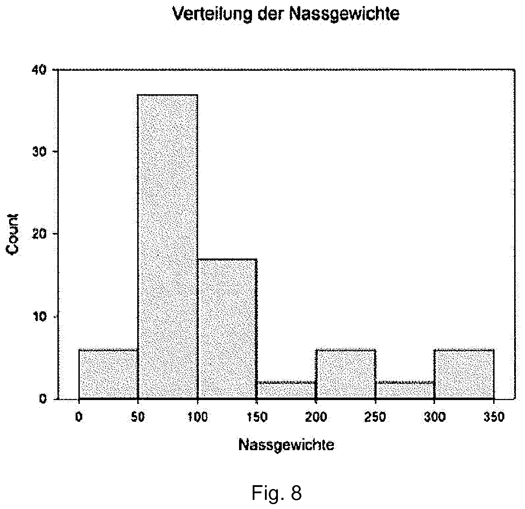

- Fig. 8: zeigt ein Histogramm zur Gewichtsverteilung der gemessenen Proben aus Knorpel, Synovia und Knochen.

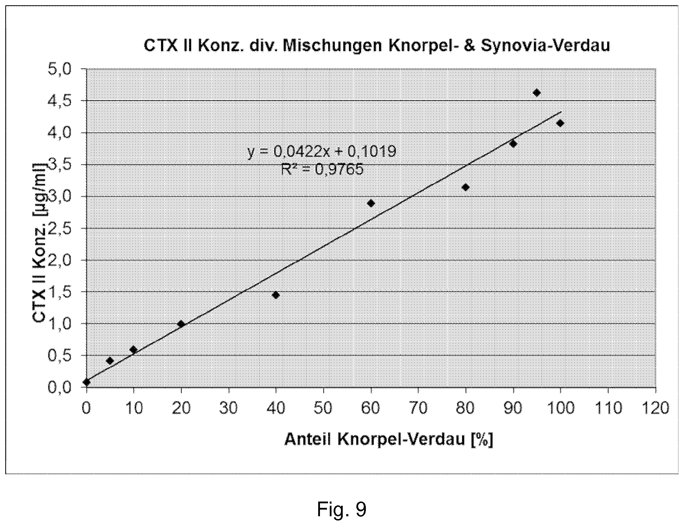

- Fig. 9: zeigt grafisch die Konzentrationen von CTX-II in diversen Mischungen eines Knorpel- und Synovialverdaus. Auf der Ordinate ist die Konzentration von CTX-II in [µg/ml] angegeben. Auf der Abszisse ist der Anteil an Knorpelverdau in [%] angegeben.

### Versuchsbeschreibung

1. Als Ausgangsmaterialien für die Verdaus wurden abgetrennte Knochenabschnitte sowie gereinigte Knorpelabschnitte von Knorpel-Knochen-Stanzen menschlicher Patienten verwendet. Außerdem wurde reines Synovialgewebe, ebenfalls von menschlichen Patienten, eingesetzt. Alle Gewebe wurden auf die gleiche Art bearbeitet und im Nassgewicht den Knorpelproben entsprechend eingesetzt.
2. Wiederfindungsversuch A
Diese Wiederfindung wurde mit Hilfe der Standards durchgeführt. Dabei wurden die Standards 1:1 verdünnt in der Verdaulösung des Fremdgewebes ("heterologe" Verdaulösung) eingesetzt: CTX-II in Synovialverdau, NTX-I in Knorpelverdau. Es sollte gezeigt werden, dass die Anwesenheit der Fremdgewebeverdaus kein Quenchen der Assays auslöst. Fig. 2 belegt, dass kein Quenching oder keine falschpositiven Werte bei diesem Versuch entstanden.
3. Wiederfindungsversuch B
In dieser Variante wurde untersucht, wie gut sich eine definierte Menge am Standard in einer homologen Verdaulösung wiederfinden lässt. Dabei wurde die Verdaulösung so verdünnt, dass sich ihr ursprünglicher Gehalt an CTX-II bzw. NTX-I messen ließ und als Parallelprobe jeweils eine mit 40 nM BCE bzw. 2.17 ng/ml CTX-II Standard gespikete, d.h. verschnittene, Probe mitgemessen wurde. Die Wiederfindungsrate wurde dann in Prozent errechnet. Nachfolgend die Statistik dazu: Die so bestimmte Wiederfindungsrate für CTX-II war 90.1 ± 1.4, die für NTX-I war 100.9 ± 15.2 %, siehe die nachfolgende Tabelle 1:

**Tabelle 1: Statistik zu den Verschnitt-Daten (Spiking-Daten)**

| Verschnitt-Daten | Größe | Durchschnitt | Standardabweichung | Standardabweichung | C.I. des Durchschnitts | C.I des Durchschnitts |
|---|---|---|---|---|---|---|
| Gewinnung von verschnittenem NTX-I (%) | 12 | 100.87887 | 15.20866 | 4.39036 | 9.66312 | |
| Verschnitt-Daten | Bereich | Max. | Min. | Median | 25% | 75 % |
| Gewinnung von verschnittenem NTX-I (%) | 56.38811 | 128.19811 | 71.81000 | 102.24872 | 89.47781 | 112.01241 |
| Verschnitt-Daten | Größe | Durchschnitt | Standardabweichung | Standardabweichung | C.I des Durchschnitts | C.I des Durchschnitts |
| Gewinnung von verschnittenem CTX-II (%) | 16 | 8 | 90.09325 | 3.96857 | 1.40310 | 3.31781 |
| Verschnitt-Daten | Bereich | Max. | Min. | Median | 25 % | 75 % |
| Gewinnung von verschnittenem CTX-II (%) | 12.22876 | 95.82523 | 83.59647 | 89.49629 | 87.80191 | 94.04336 |

4. Charakterisierung der Verdaulösungen
Es wurde die Nachweisgenauigkeit von Synovialfragmenten im Knorpelverdau bestimmt. Die oben dargestellten Wiederfindungsversuche wurden mit den in den Assaykits mitgelieferten Standards durchgeführt. Der Vorteil ist eine hohe Präzision, der Nachteil, der, dass der Kollagenaseverdau bei der Produktion nicht notwendigerweise exakt dieselben Antigenstrukturen generiert. Daher wurde als weiterer Validierungsschritt Knorpelverdau mit steigenden Anteilen an Synovialverdau gemischt und jeweils der messbare Anteil an NTX-I bestimmt. Das Ergebnis ist in der Fig. 6 dargestellt. Dabei schneidet die Regressionsgerade die Abszisse bei ca. 0.6 % vom Maximalwert des reinen Synovialverdaus. Der Regressionskoeffizient beträgt 0.977 (r²=0.9538). Daraus errechnet sich ein Bestimmungsfehler von unter 3 %, wobei er im aktuellen Fall offensichtlich bei 0.6 % lag.
In der nächsten Stufe wurden nicht Verdaus gemischt, sondern frisches Gewebe von Knorpel und Synovia gemeinsam verdaut (siehe Fig. 7). Dieses Vorgehen war allerdings mit einigen messtechnischen Problemen behaftet. So musste das Gewebestück zur Bestimmung des Nassgewichts vom überschüssigen Puffer befreit werden. Die eine Waage musste zudem steril erfolgen, um eine Kontamination der Verdaulösung zu verhindern. Als Folge dieser Maßnahmen war die Wägepräzision deutlich begrenzt und die daraus erfolgende qualitative Analyse beeinträchtigt. Trotzdem ließ sich mit diesem Verfahren eine Grobabschätzung generieren, um die Nachweisgrenze einer synovialen Kontamination zu bestimmen.
In der nachfolgenden Statistik sind die Messergebnisse einer Reihe von Gewebeverdaus präsentiert. Dabei ist jede Zelle eine Mehrfachmessung jeweils eines Spendergewebes, wobei die Mehrfachmessungen von verschiedenen Personen an zwei verschiedenen Waagen vorgenommen wurden. Die Messergebnisse von CTX-II in Synovia und Knochen sowie die Messergebnisse von NTX-I in Knorpel sind nicht dargestellt, da sie immer unter der Nachweisgrenze des jeweiligen ELISA blieben. Gemessen wurden absolute Gewebemengen von insgesamt 76 Proben im Bereich von ca. 20 mg bis 320 mg (siehe Fig. 8), wobei der Verdau jeweils in 14 ml durchgeführt wurde (entsprechend den SAA). Die Daten sind in der nachfolgenden Tabelle 2 dargestellt.

**Tabelle 2: Statistik der Crosslinkbestimmung in den Gewebeverdaus Einheiten: CTX-II: ng/ml, NTX-I: nM Knochenkollagenäquivalente**

| Verschnitt-Daten | Größe | Missing | Durchschnitt | Standardabweichung | Standardabweichung | C.I. des Durchschnitts |
|---|---|---|---|---|---|---|
| Knorpel: CTX-II/mg | 5 | 0 | 379.59929 | 66.50570 | 29.74225 | 82.57773 |
| Knorpel: CTX-II/mg | 6 | 0 | 369.42578 | 140.50912 | 57.36261 | 147.45527 |
| Synovia: NTX-I/mg | 5 | 0 | 89.71775 | 40.76337 | 18.22993 | 50.61441 |
| Synovia: NTX-I/mg | 5 | 0 | 63.03821 | 9.99895 | 4.47167 | 12.41534 |
| Knochen: NTX-I/mg | 2 | 0 | 20.49824 | 1.89793 | 1.34204 | 17.05225 |
| Knochen: NTX-I/mg | 2 | 0 | 26.36596 | 2.46503 | 1.74304 | 22.14740 |
| Verschnitt-Daten | Bereich | Max | Min | Median | 25% | 75 % |
| Knorpel: CTX-II/mg | 185.63479 | 479.74332 | 294.10853 | 372.53333 | 329.08527 | 433.64628 |
| Knorpel: CTX-II/mg | 396.62359 | 557.43614 | 160.81256 | 395.79715 | 237.48199 | 472.11394 |
| Synovia: NTX-I/mg | 93.13859 | 140.55245 | 47.41386 | 102.03846 | 47.92717 | 125.34797 |
| Synovia: NTX-I/mg | 26.12503 | 80.02303 | 53.89800 | 61.08371 | 56.07894 | 70.97473 |
| Knochen: NTX-I/mg | 2.68408 | 21.84028 | 19.15620 | 20.49824 | 19.15620 | 21.84028 |
| Knochen: NTX-I/mg | 3.48608 | 28.10900 | 24.62292 | 26.36596 | 24.62292 | 28.10900 |

Insgesamt ergab sich aus alledem für Kollagen Typ II eine Nachweisgrenze für eine unverdünnt eingesetzte Probe, für die mindestens 25 µg Knorpelgewebe benötigt werden, und für Kollagen Typ I 200 µg Synovialgewebe oder 1 mg Knochengewebe. Da in etwa 100 mg Nassgewicht als mittlere Ausgangsmenge von verarbeitetem Material zur Verfügung standen, bedeutet dies, dass immer die Identität des Knorpelgewebes festgestellt werden konnte. Das daraus abschätzbare Limit der Nachweisbarkeit von Kontamination mit Fremdgewebe beträgt für synoviales Gewebe 0.2 % und bei Knochen ca. 1 %. Bei größeren primären Einwaagen steigt die Genauigkeit entsprechend an, da die messbare Konzentration in der Verdaulösung bis auf das 3-fach (wegen bis zu 300 mg Einwaage) ansteigt. Umgekehrt sinkt die Nachweisgenauigkeit entsprechend ab, wenn nur kleine Gewebemengen zur Verarbeitung in den 14 ml Verdaulösung vorkommen. Zur Veranschaulichung: Bezogen auf den Knorpel in Fig. 1 bedeutet dies: dieser Knorpel ist real ca. 4 mm hoch gewesen, hatte also bei 4 mm Querschnitt (die Dicke der Trephine) und 4 mm Höhe (erkennbar Maßstab) ein Volumen von 48 mm³, also 48 mg. Bei dieser Einwaage wäre die Nachweisgrenze für Synovia bei 400 µg, was in etwa 0.8 % wären. Dies wäre, in Höhe umgerechnet, weniger als 40 µm Höhe, also ein Bruchteil der Höhe der superfiziellen Schicht. Eine Höhe von 40 µm entspricht in etwa der Höhe der Deckzellschicht. Bezogen auf Knochen wäre dies eine etwa 200 µm hohe Knochenschicht.
5. Bestimmung der Konzentrationen von CTX-II in diversen Mischungen eines Knorpel- und Synovialverdaus

Ein Knorpelverdau (Produktions-Probe) wurde mit aufsteigenden Volumina eines Synovialverdaus versetzt. Die Proben mussten gemäß den Vorexperimenten unterschiedlich verdünnt gemessen werden, damit sie im Bereich des Standards lagen (1:100 bis 1:3000 Verdünnungen). Die Proben-Verdünnungen mussten weiterhin in PBS gemacht werden, da nur 3 mm Standard 07 (Lösemittel) vom Hersteller mitgeliefert wurden. Um die Genauigkeit der Messung zu verbessern, wurden die Proben in Doppel-Bestimmung gemessen. Außerdem wurden die reinen Gewebeverdaus vorverdünnt, um keine Volumina kleiner 20 µl pipettieren zu müssen.

Die Bestimmung der Konzentrationen von CTX-II erfolgte mittels des ELISA-Kits Urine CartiLaps® EIA (immunodiagnosticsystems) sowie des dazugehörigen Hersteller-Protokolls.

Allgemein wurden die Inkubations-Schritte bei 20°C in einem Thermomixer inklusive Aufsatz für Temperierung von Mikrotiterplatten (Eppendorf) durchgeführt.

Zunächst wurden die Kit-Komponenten auf Raumtemperatur gebracht. Die VerdauProben wurden getaut und mit 5 mM EDTA (0.5 M EDTA 1:100 verdünnt) versetzt und gerüttelt. Danach ließ man die Proben 30 Minuten bei Raumtemperatur sedimentieren. Anschließend wurden die Proben-Verdünnungen sowie eine Mischung aus Standard-Stocklösung und Assay-Puffer angesetzt. In die Mikrovertiefungen einer mit Streptavidin vorbeschichteten ELISA-Platte (12x8 Mikrovertiefungen) wurden sodann 100 µl einer PBS-gepufferten, biotinyliertes, synthetisches Peptid (CartiLaps Antigen) enthaltenden Lösung mittels einer Mehrkanal-Präzisionspipette hinzugegeben. Daraufhin wurde die ELISA-Platte mit Klebestreifen bedeckt und während 30 Minuten bei 20 °C inkubiert (statistisch).

Es wurde ein Waschpuffer angesetzt (10 ml Waschpufferkonzentrat (50x) + 490 ml destilliertes Wasser). Die ELISA-Platte wurde 5 Mal mit 250 µl Waschpuffer gewaschen. Danach wurden 40 µl Standards, Kontrollen und Proben in die Mikrovertiefungen der ELISA-Platte pipettiert. Anschließend wurden 100 µl einer TRIS-gepufferten, einen gebrauchsfertigen monoklonalen Antikörper (Primär-Antikörper) enthaltenden Lösung in jede Mikrovertiefung der ELISA-Platte mittels einer Mehrkanal-Präzisionspipette hinzugegeben. Dann wurde die ELISA-Platte abermals mit Klebestreifen bedeckt und während 21 ± 3 Stunden bei 4 bis 8 °C inkubiert (statistisch). Daraufhin wurde die ELISA-Platte erneut 5 Mal mit 250 µl Waschpuffer gewaschen. Im Anschluss daran wurden in jede Mikrovertiefung der ELISA-Platte 100 µl einer einen Peroxidase-konjugierten Antikörper enthaltenden Lösung mittels einer Mehrkanal-Präzisionspipette hinzugegeben.

Die ELISA-Platte wurde danach erneut mit Klebestreifen bedeckt und während 60 Minuten bei 20 °C inkubiert (statistisch). Anschließend wurde die ELISA-Platte wieder 5 Mal mit 250 µl Waschpuffer gewaschen. Dann wurden in jede Mikrovertiefung der ELISA-Platte 100 µL einer chromogenen Lösung (Tetramethylbenzidin (TMB) als Substrat) mittels einer Mehrkanal-Präzisionspipette hinzugegeben. Anschließend wurde die ELISA-Platte erneut mit Klebestreifen bedeckt und während 15 Minuten bei 20 °C im Dunkeln inkubiert (statistisch). Schließlich wurden in jede Mikrovertiefung der ELISA-Platte 100 µl einer Stop-Lösung (gebrauchsfertige 0.18 mol/L Schwefelsäure) mittels einer Mehrkanal-Präzisionspipette hinzugegeben.

Die Messung der Absorption erfolgte in einem Bereich von 450 nm bis 630 nm (Platten-Reader: ELX 808 von BioTek (R 530)) innerhalb von 2 Stunden.

Die 4P Fitting der CTX-II Standard-Reihe wurde mittels der Software Gen5 1.01 von BioTek ausgewertet. Es wurden die CTX-II-Konzentrationen der Kontrollen sowie der Proben kalkuliert.

Es konnten folgende Ergebnisse erzielt werden:
Standard: gut

Absolute Abs.-Höhe ist vergleichbar mit den Angaben des Herstellerprotokolls für Urine CartiLaps® EIA. Das R² des Fittings ist mit 0.999 gut. Die Standards zeigten eine Regeneration (Recovery) zwischen 91 und 104%. Die CVs lagen zwischen 1 bis 6%. Ein Standard hatte einen CV (Varianz) von 14%.
Kontrollen: gut

Beide Kontrollproben lagen im Herstellerbereich und zeigten gute CVs.
Kontaminations-Experiment Verdauproben: gut

Der abnehmende Volumen-Anteil des Knorpelverdaus und der gleichzeitig steigende Volumen-Anteil des Synovialverdaus konnte deutlich abgebildet werden (siehe Fig. 9). Die Abstufungen waren durch die ergriffenen Maßnahmen noch deutlich herausgearbeitet.
6. Bestimmung der Konzentrationen von NTX-I in diversen Mischungen eines Knorpel- und Synovialverdaus

Ein Synovialverdau wurde mit aufsteigenden Volumina eines Knorpelverdaus (Produktionsprobe) versetzt. Die Proben mussten gemäß den Vorexperimenten unterschiedlich verdünnt gemessen werden, damit sie im Bereich des Standards lagen (1:100 bis 1:3 Verdünnungen). Eine finale 1:5 Verdünnung des Standards und der Proben ist obligatorisch. Die Proben wurden als Einzelwert gemessen.

Die Proben einer BS-Studie (Seren) wurden in Doppelbestimmung unverdünnt gemessen. Eine finale 1:5 Verdünnung des Standards und der Proben ist obligatorisch.

Die Bestimmung der NTX-I Konzentrationen erfolgte mittels des Kits OSTEOMARK® NTx Serum (Wampole Laboratories, USA; Cat.#. 9021, Lot-Nr. 13050074) sowie des dazugehörigen Hersteller-Protokolls.

Zunächst wurden die Kit-Komponenten auf Raumtemperatur gebracht. Die VerdauProben (V = 500 µl) wurden mit 5 µl 0.5 M EDTA versetzt (5 mM EDTA). Die Verdau-Kontaminations-Proben wurden angesetzt und verdünnt.

Anschließend wurden mittels eines Probenverdünnungsmediums verdünnte Proben angesetzt (2.5/1.25 nM BCE). Danach wurden die Standards, Kontrollen und Proben obligatorisch 1:5 verdünnt. Daraufhin wurde ein gereinigter monoklonaler Maus-Antikörper (gegen NTx), der mit Meerrettich-Peroxidase konjugiert war, mit Hilfe eines Antikörperkonjugat-Diluents verdünnt (12 µl Konjugat-Konzentrat (100x, CONJ) + 12 ml Konjugatverdünnungs-Medium (CONJ DIL)). Danach wurden 100 µl verdünnte Standards, Kontrollen, Proben und verdünnte Proben in die Mikrovertiefungen einer ELISA-Platte (12x8 Mikrovertiefungen) pipettiert. Die ELISA-Platte war mit adsorbiertem, synthetischen NTx-Antigen beschichtet.

Danach wurden 100 µl der verdünnten Antikörperkonjugat-Lösung (1x) in die Mikrovertiefungen der ELISA-Platte mittels einer Mehrkanal-Präzisionspipette hinzugegeben. Anschließend wurde die ELISA-Platte abgeklebt und während 20 Sek. bei 500 rpm gemischt. Danach wurde die ELISA-Platte bei 23 °C während 90 Minuten inkubiert (statistisch). Es wurde sodann ein Waschpuffer (15 ml Waschpufferkonzentrat (30x) + 435 ml destilliertes Wasser, 5 Minuten Mischen) angesetzt.

Anschließend wurde das Chromogen-Reagenz (Tetramethylbenzidin) verdünnt (200 µl Chromogen-Konzentrat (100x) + 20 ml gepuffertes Substrat (Wasserstoffperoxid)). Die ELISA-Platte wurde 5 Mal mit 300 µl Waschpuffer gewaschen. Daraufhin wurden 200 µl der Chromogen-Lösung (1 Mal) in die Mikrovertiefungen der ELISA-Platte mittels einer Mehrkanal-Präzisionspipette hinzugegeben. Dann wurde die ELISA-Platte erneut abgeklebt und bei 23 °C während 30 Minuten inkubiert (statistisch). Danach wurden in jede Mikrovertiefung der ELISA-Platte 100 µl einer Stop-Lösung (1N Schwefelsäure) zupipettiert, ehe die Platte nochmals bei 23 °C während 5 Min. inkubiert wurde.

Anschließend wurden die Extinktionswerte mittels eines Mikroplatten-Lesegerätes (ELX 808 von BioTek (R 530)) in einem Bereich von 450 nm bis 630 nm bestimmt.

Die 4P Fitting der NTX-I Standard-Reihe wurde mittels der Software Gen5 1.01 von BioTek bestimmt. Die NTX-I-Konzentrationen der Proben wurden kalkuliert.

Hierbei wurden folgende Ergebnisse erhalten:
Allgemeine Kriterien: gut
Mittlere Extinktion des 0-Standards (0 BCE): Soll: > 1.3; Ist: ca. 2.3
Extinktionsbereich des Standards (0 und 40 nM BCE): Soll: > 0.9; Ist: ca. 1.5
Standard: gut
Fitting ist gut (R² = 0.999), CVs < 5%, Regenerationen (Recoveries) 98 - 102% LLOQ:

Der Standard konnte nicht nach unten erweitert werden.
Kontrollen: ordentlich

Beide Kontroll-Proben lagen knapp unterhalb des Herstellerbereichs (4 bzw. 7%), jedoch immer noch deutlich im Vertrauens-Bereich
Verdauproben: gut

Der abnehmende Volumen-Anteil des Synovialverdaus und der gleichzeitig steigende Volumen-Anteil des Knorpelverdaus (Produktions-Probe) konnte deutlich abgebildet werden (siehe Fig. 6).
Serum-Proben: gut

In 5 von 5 unverdünnt gemessenen Proben konnte NTX-I stabil gemessen werden.

Die CVs lagen zwischen 1 bis 9%. Die NTX-I-Konzentration betrug 14.4 nM BCE (± 2.7) (Bereich: 10.9 bis 19.3)

## Patentansprüche

1. Verfahren zum in vitro Nachweis von Knorpelgewebe und/oder zur in vitro Bestimmung der Reinheit von Knorpelgewebe, umfassend die folgenden Schritte:
a) Behandeln einer Gewebeprobe mit einer Protease und
b) Untersuchen der Protease-behandelten Gewebeprobe auf die Anwesenheit von Protease-resistenten Fragmenten von Kollagen Typ II und/oder Kollagen Typ I, **dadurch gekennzeichnet, dass** es sich bei den Protease-resistenten Fragmenten von Collagen Typ II um C-terminale Collagenfragmente und bei den Protease-resistenten Fragmenten von Collagen Typ I um N-terminale Collagenfragmente handelt oder umgekehrt.

2. Verfahren nach Anspruch 1, **dadurch gekennzeichnet, dass** es sich bei der Gewebeprobe um ein Gelenkbiopsat, insbesondere Kniegelenkbiopsat, handelt.

3. Verfahren nach Anspruch 1 oder 2, **dadurch gekennzeichnet, dass** vor Durchführen von Schritt a) superfizielles Knorpelgewebe, mineralisiertes Knorpelgewebe und/oder Knochengewebe, insbesondere subchondrales Knochengewebe, aus der Gewebeprobe entfernt wird.

4. Verfahren nach einem der vorhergehenden Ansprüche, **dadurch gekennzeichnet, dass** es sich bei der Gewebeprobe um ein Gewebefragment, insbesondere Knorpelgewebefragment, eines Knorpel-Knochen-Stanzbiopsats handelt.

5. Verfahren nach einem der vorhergehenden Ansprüche, **dadurch gekennzeichnet, dass** es sich bei der Protease um Kollagenase, bevorzugt bakterielle Kollagenase, handelt.

6. Verfahren nach einem der vorhergehenden Ansprüche, **dadurch gekennzeichnet, dass** es sich bei den Protease-resistenten Fragmenten von Kollagen Typ II und/oder Kollagen Typ I um quernetzte Kollagenfragmente handelt.

7. Verfahren nach einem der vorhergehenden Ansprüche, **dadurch gekennzeichnet, dass** die Protease-resistenten Fragmente von Kollagen Typ II und/oder Kollagen Typ I Quervernetzungen aufweisen, welche aus der Gruppe, umfassend Allysin-Quervernetzungen, Hydroxyallysin-Quervernetzungen, Dehydro-hydroxylysino-norleucin-Quervernetzungen, Dehydro-lysino-norleucin-Quervernetzungen, Hydroxy-lysino-5-ketonorleucin-Quervernetzungen, Dihydroxy-lysino-norleucin-Quervernetzungen, Histidino-hydroxylysino-norleucin-Quervernetzungen, Hydroxylysylpyridinolin-Quervernetzungen, Lysylpyridinolin-Quervernetzungen, Pyridinium-Quervernetzungen, 3-Hydroxypyridinium-Quervernetzungen, Histidinohydroxymerodesmosin-Quervernetzungen und Kombinationen davon, ausgewählt sind.

8. Verfahren nach einem der vorhergehenden Ansprüche, **dadurch gekennzeichnet, dass** es sich bei den Protease-resistenten Fragmenten von Kollagen Typ II um C-terminale quervernetzte Typ II Kollagen Telopeptide (CTX-II) oder Fragmente davon handelt.

9. Verfahren nach einem der vorhergehenden Ansprüche, **dadurch gekennzeichnet, dass** es sich bei den Protease-resistenten Fragmenten von Kollagen Typ I um N-terminale quervernetzte Typ I Kollagen Telopeptide (NTX-I) oder Fragmente davon handelt.

10. Verfahren nach einem der vorhergehenden Ansprüche, **dadurch gekennzeichnet, dass** beim Durchführen von Schritt b) die Protease-behandelte Gewebeprobe mit einem Antikörper oder Antikörperfragment kontaktiert wird, wobei der Antikörper bzw. das Antikörperfragment spezifisch gegen Protease-resistente Fragmente von Kollagen Typ II gerichtet ist.

11. Verfahren nach einem der vorhergehenden Ansprüche, **dadurch gekennzeichnet, dass** beim Durchführen von Schritt b) die Protease-behandelte Gewebeprobe mit einem Antikörper oder Antikörperfragment kontaktiert wird, wobei der Antikörper bzw. das Antikörperfragment spezifisch gegen Protease-resistente Fragmente von Kollagen Typ I gerichtet ist.

12. Verfahren nach Anspruch 10 oder 11, **dadurch gekennzeichnet, dass** die Bindung des Antikörpers bzw. Antikörperfragments an die Protease-resistenten Fragmente mittels einer immunologischen Technik, vorzugsweise mittels ELISA-Technik, detektiert wird.

13. Verfahren zur Herstellung einer Knorpelzellkultur, umfassend die folgenden Schritte:
a) Behandeln einer Knorpelgewebeprobe mit einer Protease,
b) Untersuchen der Protease-behandelten Knorpelgewebeprobe auf die Anwesenheit von Protease-resistenten Fragmenten von Kollagen Typ II und/oder Kollagen Typ I und
c) Kultivieren von in der untersuchten Knorpelgewebeprobe enthaltenen Knorpelzellen, **dadurch gekennzeichnet, dass** es sich bei den Protease-resistenten Fragmenten von Collagen Typ II um C-terminale Collagenfragmente und bei den Protease-resistenten Fragmenten von Collagen Typ I um N-terminale Collagenfragmente handelt oder umgekehrt.

14. Verfahren zur Herstellung eines knorpelzellbeladenen Implantats, insbesondere zur Verwendung bei der Matrix-gestützten Chondrozytentransplantation (MACT), umfassend die folgenden Schritte:
a) Behandeln einer Knorpelgewebeprobe mit einer Protease,
b) Untersuchen der Protease-behandelten Knorpelgewebeprobe auf die Anwesenheit von Protease-resistenten Fragmenten von Kollagen Typ II und/oder Kollagen Typ I,
c) Kultivieren von in der untersuchten Knorpelgewebeprobe enthaltenen Knorpelzellen und
d) Beladen eines Implantats mit den kultivierten Knorpelzellen, **dadurch gekennzeichnet, dass** es sich bei den Protease-resistenten Fragmenten von Collagen Typ II um C-terminale Collagenfragmente und bei den Protease-resistenten Fragmenten von Collagen Typ I um N-terminale Collagenfragmente handelt oder umgekehrt.

15. Verwendung von Protease-resistenten Fragmenten von Kollagen Typ II und/oder Kollagen Typ I zum in vitro Nachweis von Knorpelgewebe und/oder zur in vitro Bestimmung der Reinheit von Knorpelgewebe, **dadurch gekennzeichnet, dass** es sich bei den Protease-resistenten Fragmenten von Collagen Typ II um C-terminale Collagenfragmente und bei den Protease-resistenten Fragmenten von Collagen Typ I um N-terminale Collagenfragmente handelt oder umgekehrt.

## Claims

1. Method for the in vitro detection of cartilage tissue and/or for the in vitro determination of the purity of cartilage tissue, comprising the following steps:
a) treating a tissue sample with a protease and
b) testing the protease-treated tissue sample for the presence of protease-resistant fragments of type II collagen and/or type I collagen,
**characterized in that**
the protease-resistant fragments of type II collagen are C-terminal collagen fragments and the protease-resistant fragments of type I collagen are N-terminal collagen fragments or vice versa.

2. Method according to claim 1, **characterized in that** the tissue sample is a joint biopsy, more particularly a knee joint biopsy.

3. Method according to claim 1 or 2, **characterized in that** superficial cartilage tissue, mineralized cartilage tissue and/or bone tissue, more particularly subchondral bone tissue, is removed from the tissue sample before step a) is carried out.

4. Method according to any of the preceding claims, **characterized in that** the tissue sample is a tissue fragment, more particularly a cartilage tissue fragment, of a cartilage/bone core biopsy.

5. Method according to any of the preceding claims, **characterized in that** the protease is collagenase, preferably bacterial collagenase.

6. Method according to any of the preceding claims, **characterized in that** the protease-resistant fragments of type II collagen and/or type I collagen are crosslinked collagen fragments.

7. Method according to any of the preceding claims, **characterized in that** the protease-resistant fragments of type II collagen and/or type I collagen have crosslinks selected from the group comprising allysine crosslinks, hydroxyallysine crosslinks, dehydrohydroxylysinonorleucine crosslinks, dehydrolysinonorleucine crosslinks, hydroxylysino-5-ketonorleucine crosslinks, dihydroxylysinonorleucine crosslinks, histidinohydroxylysinonorleucine crosslinks, hydroxylysylpyridinoline crosslinks, lysylpyridinoline crosslinks, pyridinium crosslinks, 3-hydroxypyridinium crosslinks, histidinohydroxymerodesmosine crosslinks and combinations thereof.

8. Method according to any of the preceding claims, **characterized in that** the protease-resistant fragments of type II collagen are C-terminal crosslinked type II collagen telopeptides (CTX-II) or fragments thereof.

9. Method according to any of the preceding claims, **characterized in that** the protease-resistant fragments of type I collagen are N-terminal crosslinked type I collagen telopeptides (NTX-I) or fragments thereof.

10. Method according to any of the preceding claims, **characterized in that** step b) is carried out by contacting the protease-treated tissue sample with an antibody or antibody fragment, the antibody or the antibody fragment being specifically directed against protease-resistant fragments of type II collagen.

11. Method according to any of the preceding claims, **characterized in that** step b) is carried out by contacting the protease-treated tissue sample with an antibody or antibody fragment, the antibody or the antibody fragment being specifically directed against protease-resistant fragments of type I collagen.

12. Method according to claim 10 or 11, **characterized in that** an immunological technique, preferably ELISA technique, is used to detect the binding of the antibody or antibody fragment to the protease-resistant fragments.

13. Method for preparing a cartilage cell culture, comprising the following steps:
a) treating a cartilage tissue sample with a protease,
b) testing the protease-treated cartilage tissue sample for the presence of protease-resistant fragments of type II collagen and/or type I collagen, and
c) culturing cartilage cells present in the tested cartilage tissue sample, **characterized in that** the protease-resistant fragments of type II collagen are C-terminal collagen fragments and the protease-resistant fragments of type I collagen are N-terminal collagen fragments or vice versa.

14. Method for preparing a cartilage cell-loaded implant, more particularly for use in matrix-assisted chondrocyte transplantation (MACT), comprising the following steps:
a) treating a cartilage tissue sample with a protease,
b) testing the protease-treated cartilage tissue sample for the presence of protease-resistant fragments of type II collagen and/or type I collagen,
c) culturing cartilage cells present in the tested cartilage tissue sample and
d) loading an implant with the cultured cartilage cells, **characterized in that** the protease-resistant fragments of type II collagen are C-terminal collagen fragments and the protease-resistant fragments of type I collagen are N-terminal collagen fragments or vice versa.

15. Use of protease-resistant fragments of type II collagen and/or type I collagen for the in vitro detection of cartilage tissue and/or for the in vitro determination of the purity of cartilage tissue, **characterized in that** the protease-resistant fragments of type II collagen are C-terminal collagen fragments and the protease-resistant fragments of type I collagen are N-terminal collagen fragments or vice versa.

## Revendications

1. Procédé pour la détection in vitro de tissu cartilagineux et/ou pour la détermination in vitro de la pureté de tissu cartilagineux, comportant les étapes suivantes :
a) traiter un échantillon de tissu avec une protéase et
b) analyser l'échantillon de tissu traité de protéase pour la présence de fragments résistants aux protéases de collagène de type II et/ou de collagène de type I, **caractérisé en ce que** les fragments résistants aux protéases de collagène de type II sont des fragments de collagène C-terminal et les fragments résistants aux protéases de collagène de type I sont des fragments de collagène N-terminal ou vice versa.

2. Procédé selon la revendication 1, **caractérisé en ce que** l'échantillon de tissu est une biopsie d'articulation, en particulier une biopsie d'articulation du genou.

3. Procédé selon la revendication 1 ou 2, **caractérisé en ce que** du tissu cartilagineux superficiel, du tissu cartilagineux minéralisé et/ou du tissu osseux, en particulier du tissu osseux sous-chondral, sont éliminés de l'échantillon de tissu avant d'exécuter l'étape a).

4. Procédé selon l'une quelconque des revendications précédentes, **caractérisé en ce que** l'échantillon de tissu est un fragment de tissu, en particulier un fragment de tissu cartilagineux, d'une biopsie par forage de cartilage-os.

5. Procédé selon l'une quelconque des revendications précédentes, **caractérisé en ce que** la protéase est de la collagénase, de préférence collagénase bactérienne.

6. Procédé selon l'une quelconque des revendications précédentes, **caractérisé en ce que** les fragments résistants aux protéases de collagène de type II et/ou de collagène de type I sont des fragments de collagène réticulés.

7. Procédé selon l'une quelconque des revendications précédentes, **caractérisé en ce que** les fragments résistants aux protéases de collagène de type II et/ou de collagène de type I comportent des réticulations transversales sélectionnées parmi le groupe consistant en réticulations transversales d'allysine, réticulations transversales de hydroallysine, réticulations transversales de déhydro-hydroxylysino-norleucine, réticulations transversales de déhydro-lysino-norleucine, réticulations transversales de hydroxy-lysino-5-kétonorleucine, réticulations transversales de dihydroxy-lysino-norleucine, réticulations transversales de histidino-hydroxylysino-norleucine, réticulations transversales de hydroxylysylpyridinoline, réticulations transversales de lysylpyridinoline, réticulations transversales de pyridinium, réticulations transversales de 3-hydroxypyridinium, réticulations transversales de histidino-hydroxymerodesmosine et des combinaisons de ceux-ci.

8. Procédé selon l'une quelconque des revendications précédentes, **caractérisé en ce que** les fragments résistants aux protéases de collagène de type II sont des télopeptides de collagène de type II C-terminal réticulés (CTX-II) ou des fragments de ceux-ci.

9. Procédé selon l'une quelconque des revendications précédentes, **caractérisé en ce que** les fragments résistants aux protéases de collagène de type I sont des télopeptides de collagène de type I N-terminal réticulés (NTX-I) ou des fragments de ceux-ci.

10. Procédé selon l'une quelconque des revendications précédentes, **caractérisé en ce que** l'exécution de l'étape b) se fait en contactant le tissu traité de protéase avec un anticorps ou un fragment d'anticorps, dans lequel l'anticorps ou le fragment d'anticorps est dirigé spécifiquement contre des fragments résistants aux protéases de collagène de type II.

11. Procédé selon l'une quelconque des revendications précédentes, **caractérisé en ce que** l'exécution de l'étape b) se fait en contactant le tissu traité de protéase avec un anticorps ou un fragment d'anticorps, dans lequel l'anticorps ou le fragment d'anticorps est dirigé spécifiquement contre des fragments résistants aux protéases de collagène de type I.

12. Procédé selon la revendication 10 ou 11, **caractérisé en ce que** la liaison de l'anticorps ou du fragment d'anticorps aux fragments résistants aux protéases est détectée au moyen d'une technique immunologique, de préférence la technique ELISA.

13. Procédé pour la préparation d'une culture de cellules cartilagineuses, comportant les étapes suivantes :
a) traiter un échantillon de tissu cartilagineux avec une protéase,
b) analyser l'échantillon de tissu cartilagineux traité de protéase pour la présence de fragments résistants aux protéases de collagène de type II et/ou de collagène de type I et
c) cultiver des cellules cartilagineuses présentes dans l'échantillon de tissu cartilagineux analysé, **caractérisé en ce que** les fragments résistants aux protéases de collagène de type II sont des fragments de collagène C-terminal et les fragments résistants aux protéases de collagène de type I sont des fragments de collagène N-terminal ou vice versa.

14. Procédé pour la préparation d'un implant chargé de cellules cartilagineuses, en particulier pour l'utilisation dans la transplantation de chondrocytes assistée par matrices (MACT), comportant les étapes suivantes :
a) traiter un échantillon de tissu cartilagineux avec une protéase,
b) analyser l'échantillon de tissu cartilagineux traité de protéase pour la présence de fragments résistants aux protéases de collagène de type II et/ou de collagène de type I,
c) cultiver des cellules cartilagineuses présentes dans l'échantillon de tissu cartilagineux analysé et
d) charger un implant avec les cellules cartilagineuses cultivées, **caractérisé en ce que** les fragments résistants aux protéases de collagène de type II sont des fragments de collagène C-terminal et les fragments résistants aux protéases de collagène de type I sont des fragments de collagène N-terminal ou vice versa.

15. Utilisation de fragments résistants aux protéases de collagène de type II et/ou de collagène de type I pour la détection in vitro de tissu cartilagineux et/ou pour la détermination in vitro de la pureté de tissu cartilagineux, **caractérisée en ce que** les fragments résistants aux protéases de collagène de type II sont des fragments de collagène C-terminal et les fragments résistants aux protéases de collagène de type I sont des fragments de collagène N-terminal ou vice versa.
